# EUROPEAN PATENT SPECIFICATION

(11) **EP 1 641 756 B1**
(45) Date of publication and mention of the grant of the patent: **11.04.2007**
(21) Application number: 04731651.8
(22) Date of filing: 07.05.2004
(51) Int. Cl.: C07D 211/54, C07D 417/06, C07D 401/12, C07D 413/12, A61K 31/445, A61P 25/00

(54) **4-ARYLSULPHONYLPIPERIDINE DERIVATIVES FOR ANTAGONISM OF THE 5-HT2A RECEPTOR**
4-ARYLSULPHONYLPIPERIDIN-DERIVATE ALS 5-HT2A -REZEPTOR ANTAGONISTEN
DERIVES DE 4-ARYLSULPHONYLPIPERIDINE POUR L'ANTAGONISME DU RECEPTEUR DE 5-HT2A

(30) Priority: 16.05.2003 GB 0311349
(43) Date of publication of application: 05.04.2006
(73) Proprietor: MERCK SHARP & DOHME LTD., Hoddesdon, Hertfordshire EN11 9BU (GB)
(72) Inventor: GILLIGAN, Myra, Harlow, Essex CM20 2QR (GB); HUMPHRIES, Alexander, Charles, Harlow, Essex CM20 2QR (GB); LADDUWAHETTY, Tamara, Harlow, Essex CM20 2QR (GB)
(74) Representative: Buchan, Gavin MacNicol
(86) International application number: PCT/GB2004/001998
(87) International publication number: WO 2004/101518

(56) References cited:
- WO-A-00/43362
- WO-A-01/51469
- S. R. FLETCHER ET AL.: "4-(Phenylsulfonyl)piperidines: Novel, Selective, and Bioavailable 5-HT2A Receptor antagonists" J. MED. CHEM., vol. 45, no. 2, 2002, XP002290071 cited in the application

## Description

The present invention relates to a class of sulphonyl derivatives which act on serotonin receptors (also known as 5-hydroxytryptamine or 5-HT receptors). More particularly, the invention concerns 4-arylsulphonylpiperidine derivatives which bear a fluorine substituent in the 4-position. These compounds are potent and selective antagonists of the human 5-HT_{2A} receptor and are therefore useful as pharmaceutical agents, especially in the treatment and/or prevention of adverse conditions of the central nervous system, including sleep disorders such as insomnia, psychotic disorders such as schizophrenia and psychiatric disorders such as anxiety.

Compounds of the invention typically display more effective binding to the human 5-HT_{2A} receptor than to other human receptors such as D₂, 5HT_{2C} and IKr receptors. They can therefore be expected to manifest fewer side-effects than compounds which do not discriminate in their binding affinity between such receptors. In particular these compounds have lower effects on the IKr receptors and there is a separation of the desired effect from side effects such as cardiac effects.

By virtue of their potent human 5-HT_{2A} receptor antagonist activity, the compounds of the present invention are effective in the treatment of neurological conditions including sleep disorders such as insomnia, psychotic disorders such as schizophrenia, and also depression, anxiety, panic disorder, obsessive-compulsive disorder, pain, eating disorders such as anorexia nervosa, and dependency or acute toxicity associated with narcotic agents such as LSD or MDMA; and moreover are beneficial in controlling the extrapyramidal symptoms associated with the administration of neuroleptic agents. They may further be effective in the lowering of intraocular pressure, and may also be effective in treating menopausal symptoms, in particular hot flushes (see Waldinger et al, *Maturitas,* 2000, **36**, 165-8).

Various classes of compounds containing *inter alia* a sulphonyl moiety are described in WO 00/43362, WO 96/35666, EP-A-0261688, EP-0304888, and US Patents 4,218,455 and 4,128,552, DE-A-3901735 and Fletcher *et al, J. Med. Chem.,* 2002, **45**, 492-503. None of these publications, however, discloses or suggests the particular class of compounds provided by the present invention.

The compounds according to the present invention are potent and selective 5-HT_{2A} receptor antagonists, typically having a human 5-HT_{2A} receptor binding affinity (Kᵢ) of 100 nM or less, typically of 50 nM or less and preferably of 10 nM or less. The compounds of the invention may possess at least a 1 0-fold selective affinity, suitably at least a 20-fold selective affinity and preferably at least a 50-fold selective affinity, for the human 5-HT_{2A} receptor relative to the human dopamine D₂ receptor. The compounds of this invention may possess at least a 1 0-fold selective affinity, suitably at least a 20-fold selective affinity and preferably at least a 50-fold selective affinity of the human 5-HT_{2A} receptor relative to the IKr. The compounds of this invention may possess at least a 1 0-fold selective affinity, suitably at least a 20-fold selective affinity and preferably at least a 50-fold selective affinity for the human 5-HT_{2A} receptor relative to the human 5-HT_{2c} receptor. Preferred compounds show selectivities of at least 100 fold relative to the human 5-HT_{2c} receptor.

The present invention provides a compound of formula I: 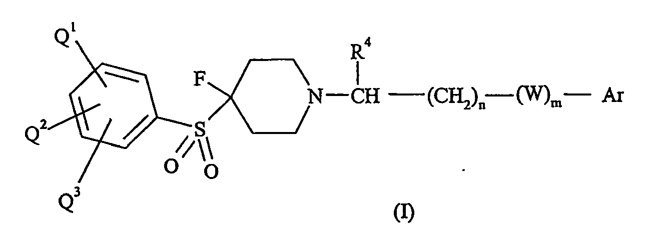 or a pharmaceutically acceptable salt thereof wherein:
Ar is phenyl, benzisothiazol-3-yl or benzthiophen-3-yl, each of which bears substituent groups R¹, R² and R³;
R¹ is hydrogen, fluorine, chlorine, bromine, C₁₋₆ alkyl, C₃₋₆ cycloalkyl, C₂₋₆ alkenyl, C₂₋₆ alkynyl, C₁₋₆ alkoxy, C₂₋₆ alkenyloxy, C₂₋₆ alkynyloxy, or C₁₋₆ alkyl substituted by up to 5-fluorine atoms;
R² is hydrogen, fluorine, chlorine, C₁₋₄ alkyl, C₁₋₄ alkoxy, C₁₋₄ alkyl substituted by up to 5 fluorine atoms or C₁₋₄ alkoxy substituted by up to 5 fluorine atoms;
R³ is hydrogen, fluorine, chlorine, methyl, methoxy, trifluoromethyl, difluoromethyl, trifluoromethoxy or difluoromethoxy;
Q¹ is hydrogen; fluorine; chlorine; bromine; C₁₋₆ alkyl; C₃₋₆ cycloalkyl; C₂₋₆ alkenyl; C₂₋₆ alkynyl; C₁₋₆ alkoxy; C₂₋₆ alkenyloxy; C₂₋₆ alkynyloxy; C₁₋₆ alkyl substituted by up to 5-fluorine atoms; nitrile; COQ⁴ or CO₂Q⁴ where Q⁴ is hydrogen or C₁₋₆ alkyl; NQ⁵Q⁶, CONQ⁵Q⁶ or SO₂NQ⁵Q⁶ where Q⁵ is hydrogen or C₁₋₆ alkyl and Q⁶ is hydrogen or C₁₋₆ alkyl or Q⁵ and Q⁶ are joined to form either a 4-7 membered heterocyclic ring which may also contain one oxygen or one further nitrogen ring atom, which heterocyclic ring may optionally be substituted by up to 3 fluorine atoms or by CF₃, methyl, ethyl or hydroxyl; hydroxyl; nitro; SOQ⁷ or S0_{2Q}⁷ where Q⁷ is C₁₋₄ alkyl; NQ⁸COQ⁹, NQ⁸CO₂Q⁹ or NQ⁸SO₂Q⁹ where Q⁸ is hydrogen or C₁₋₄alkyl and Q⁹ is hydrogen or C₁₋₄alkyl or is joined to Q⁸ to form a 5-7 membered ring; a heteroaromatic ring of 5 ring atoms 1, 2, 3 or 4 of which maybe nitrogen atoms or 1 or 2 of which are nitrogen atoms and 1 of which is an oxygen or sulfur atom or 1 of which is an oxygen or sulfur atom, which heteroaromatic ring optionally being substituted by methyl, ethyl or hydroxyl; or a heteroaromatic ring of 6 ring atoms containing 1 or 2 nitrogen ring atoms or a phenyl group either of which is optionally substituted by 1 or 2 fluorine or chlorine atoms or C₁₋₄alkyl, C₁₋₄alkoxy or trifluoromethyl groups;
Q² is hydrogen, fluorine, chlorine, nitrile, hydroxy, C₁₋₄ alkyl, C₁₋₄ alkoxy, C₁₋₄ alkyl substituted by up to 5 fluorine atoms, or C₁₋₄ alkoxy substituted by up to 5 fluorine atoms;
Q³ is hydrogen, fluorine, chlorine, methyl, methoxy, trifluoromethyl, difluoromethyl, trifluoromethoxy or difluoromethoxy;
or Q² and Q³ are joined to form the residue of a 5, 6 or 7 membered carbocyclic ring;
R⁴ is H or C₁₋₄ alkyl,
m is 0 or 1;
n is 0, 1 or 2; and
W is CH₂, CHF, CH(OH) or CO.

In a subset of the compounds of formula I, R⁴ is H, m is 1, n is 0, W is CH₂, Ar is phenyl bearing the substituent groups R¹, R² and R³, and heterocyclic rings represented by NQ⁵Q⁶ are optionally substituted by methyl, ethyl or hydroxyl.

Suitable C₁₋₆ alkyl groups can be straight or branched. Hence such groups include methyl, ethyl, n-propyl, isopropyl, n-butyl, isobutyl, secbutyl, and straight and branched pentyl and hexyl groups.

Favoured C₁₋₆ alkyl groups include methyl, ethyl, n-propyl, and the like.

A preferred C₁₋₆ alkyl group is the methyl group.

Suitable and favoured alkenyl and alkynyl groups are analogous to the suitable and favoured alkyl groups.

The expression "C₃₋₆cycloalkyl" as used herein refers to nonaromatic monocyclic hydrocarbon ring systems comprising from 3 to 6 ring atoms. Examples include cyclopropyl, cyclobutyl, cyclopentyl, cyclohexyl and cyclohexenyl.

For use in medicine, the compounds of formula I may be in the form of pharmaceutically acceptable salts. Other salts may, however, be useful in the preparation of the compounds of formula I or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, benzenesulphonic acid, fumaric acid, maleic acid, succinic acid, acetic acid, benzoic acid, oxalic acid, citric acid, tartaric acid, carbonic acid or phosphoric acid. Alternatively, where the compound of the invention carries an acidic moiety, the compound of formula I may exist as a zwitterion, or a pharmaceutically acceptable salt may be formed by neutralisation of said acidic moiety with a suitable base. Examples of pharmaceutically acceptable salts thus formed include alkali metal salts such as sodium or potassium salts; ammonium salts; alkaline earth metal salts such as calcium or magnesium salts; and salts formed with suitable organic bases, such as amine salts (including pyridinium salts) and quaternary ammonium salts. The compounds of formula I are typically in the form of the free base, the hydrochloride salt or the methanesulfonate salt.

When the compounds according to the invention have one or more asymmetric centres, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

In the compounds of formula I, R⁴ represents H or C₁₋₄ alkyl (such as methyl, ethyl or propyl), preferably H or methyl, and most preferably H.
n represents 0, 1 or 2, preferably 0 or 1, and most preferably 0.
m represents 0 or 1, typically 1 except when Ar represents a benzisothiazole or benzthiophene residue, in which case n and m are preferably both 0.
W represents CH₂, CHF, CH(OH) or CO, preferably CH₂, CHF or CO. In a particular embodiment, m=1, W represents CH₂, n=0 and R⁴ is H.
Q¹ is preferably located para to the SO₂ group. Preferred identities for Q¹ include H, F, Cl, Br, CN, carboxamide, 5-membered heteroaryl and NQ⁵Q⁶ where Q⁵ and Q⁶ preferably complete a heterocyclic ring as defined previously. Examples of 5-membered heteroaryl rings represented by Q¹ include pyrrole, pyrazole, imidazole, triazole, tetrazole, oxazole and thiazole. Such rings may be bonded through carbon or alternatively, in the case of pyrrole, pyrazole, imidazole, triazole and tetrazole, through nitrogen. Examples of heterocyclic groups represented by NQ⁵Q⁶ include azetidinyl, pyrrolidinyl, piperidinyl, morpholinyl, 4-trifluoromethylpiperidin-1-yl and 4,4-difluoropiperdin-1-yl.
Q² is preferably H, F, Cl, CF₃, methyl or methoxy, especially H, F or Cl, and is most preferably H or F.
Q³ is preferably H or F, most preferably H.
R¹ is preferably attached in the 4-position when Ar represents a phenyl residue. Preferred identities for R¹ include H, F, Cl, Br, C₁₋₆ alkyl (such as methyl), methoxy and CF₃, in particular H, F, methyl and CF₃.
R² is preferably attached in the 2-position when Ar represents a phenyl residue. Preferred identities for R² include H, F, Cl, C₁₋₄alkyl (such as methyl) and CF₃, in particular H, F, methyl and CF₃.
R³ is preferably H or F, most preferably H.

Certain favoured compounds of the invention include those of formula (II): 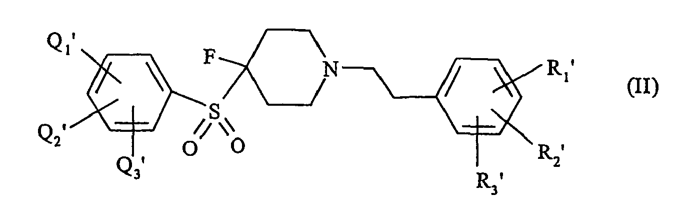 and pharmaceutically acceptable salts thereof wherein:
R_{1'} is hydrogen, fluorine, chlorine, methyl, trifluoromethyl or methoxy;
R_{2'} is hydrogen, fluorine or chlorine;
R_{3'} is hydrogen or fluorine;
Q_{1'} is hydrogen, fluorine, chlorine, bromine, methyl, trifluoromethyl, methoxy, carboxamide, nitrile, or 1,2,3-triazolyl;
Q_{2'} is hydrogen, fluorine or chlorine;
Q_{3'} is hydrogen or fluorine.

In certain preferred compounds of formula (II) R_{1'} is hydrogen or fluorine, R_{2'} is hydrogen or fluorine and R_{3'} is hydrogen.

In certain preferred compounds of formula (II) Q_{1'} is at the 4-position and is hydrogen, fluorine, carboxamide, nitrile or 1,2,3-triazolyl, Q_{2'} is hydrogen or fluorine and Q_{3'} is hydrogen.

A preferred subset of the compounds of the invention comprises the compounds of formula IIA: 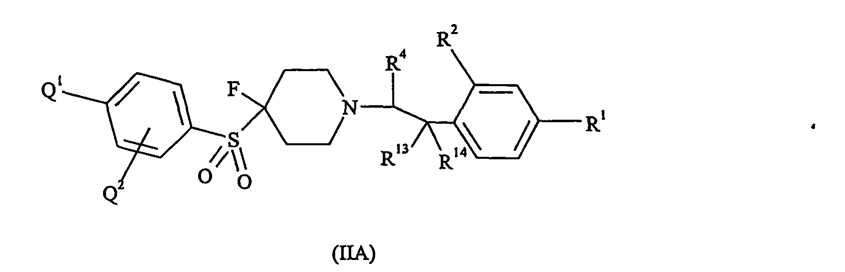 and pharmaceutically acceptable salts thereof;
wherein R¹³ represents H and R¹⁴ represents H, F or OH, or R¹³ and R¹⁴ together represent keto;
and Q¹, Q², R¹, R² and R⁴ have the same meanings and preferred identities as before.

Within this embodiment, R¹ and R² are very suitably independently selected from H and F.

Within this embodiment, Q² is preferably H or F.

Within this embodiment, preferred identities for Q¹ include H, F, Cl, Br, CN, CONH₂, morpholin-1-yl, 1,2,3-triazol-1-yl, 1,2,4-triazol-1-yl, pyrazol-1-yl, imidazole-1-yl, pyrrol-1-yl, 1-methylpyrazol-5-yl, 1-methylimidazol-2-yl, 2-methyltetrazol-5-yl, oxazol-2-yl, thiazol-2-yl, azetidin-1-yl, pyrrolidin-1-yl, piperidin-1-yl, 4,4-difluoro-piperidin-1-yl and 4-(trifluoromethyl)piperidin-1-yl.

Specific compounds of this invention include those compounds exemplified hereinafter and their pharmaceutically acceptable salts.

The compounds of the present invention have an activity as antagonists of the human 5-HT_{2A} receptor and hence find use in the treatment or prevention of disorders mediated by 5-HT_{2A} receptor activity.

The invention also provides pharmaceutical compositions comprising one or more compounds of this invention and a pharmaceutically acceptable carrier. Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. The principal active ingredient typically is mixed with a pharmaceutical carrier, e.g. conventional tableting ingredients such as corn starch, lactose, sucrose, sorbitol, talc, stearic acid, magnesium stearate and dicalcium phosphate, or gums, dispersing agents, suspending agents or surfactants such as sorbitan monooleate and polyethylene glycol, and other pharmaceutical diluents, e.g. water, to form a homogeneous preformulation composition containing a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. Tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, liquid- or gel-filled capsules, suitably flavoured syrups, aqueous or oil suspensions, and flavoured emulsions with edible oils such as cottonseed oil, sesame oil or coconut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, poly(ethylene glycol), poly(vinylpyrrolidone) or gelatin.

The present invention also provides a compound of formula I or a pharmaceutically acceptable salt thereof for use in a method of treatment of the human body. Preferably the treatment is for a condition mediated by 5-HT_{2A} receptor activity.

The present invention further provides the use of a compound of formula I or a pharmaceutically acceptable salt thereof in the manufacture of a medicament for treating or preventing a condition mediated by 5-HT_{2A} receptor activity.

Also disclosed is a method of treatment of a subject suffering from or prone to a condition mediated by 5-HT_{2A} receptor activity which comprises administering to that subject an effective amount of a compound according to formula I or a pharmaceutically acceptable salt thereof.

In one aspect of the invention, the condition mediated by 5-HT_{2A} receptor activity is sleep disorder, in particular insomnia. In a further aspect of the invention, the condition mediated by 5-HT_{2A} receptor activity is selected from psychotic disorders (such as schizophrenia), depression, anxiety, panic disorder, obsessive-compulsive disorder, pain, eating disorders (such as anorexia nervosa), dependency or acute toxicity associated with narcotic agents such as LSD or MDMA, and hot flushes associated with the menopause.

In the treatment envisaged herein, for example of insomnia or schizophrenia, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day but preferably once per day, for example before going to bed.

If desired, the compounds according to this invention may be co-administered with another sleep inducing or anti-schizophrenic or anxiolytic medicament. Such coadministration may be desirable where a patient is already established on sleep inducing or anti-schizophrenic or anxiolytic treatment regime involving other conventional-medicaments.

The compounds of the formula (I) may be prepared by the reaction of the compounds of the formulae (III) and (IV): 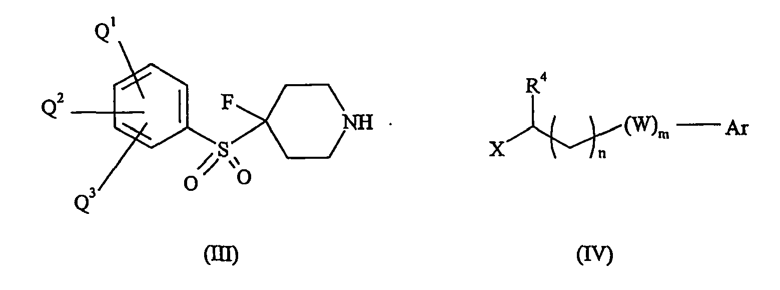 where X is a leaving group (especially halide, such as Br) and all other variables have the same meanings as before. The reaction takes place under conventional reaction conditions for alkylation, e.g. using a solvent such as tetrahydrofuran or acetonitrile at a temperature such as 80°C to 90°C in the presence of a base such as K₂CO₃, AgO or CsCO₃. A catalytic quantity of NaI may be employed in the conventional manner.

Alternatively, compounds of formula (III) may be reductively alkylated with compounds of formula (IVA): 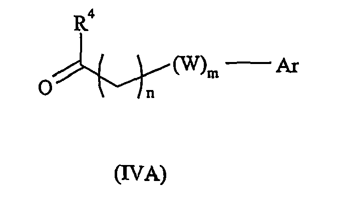 where Ar, m, n, W and R⁴ are as defined previously (but W is preferably CH₂). The reaction takes place in ethanol in the presence of sodium borohydride and titanium tetraisopropoxide.

Compounds of formula (III) may alternatively be reacted with epoxides (IVB): 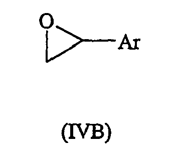 where Ar is as defined previously to provide compounds of formula (I) wherein R⁴ is H, n is 0, m is 1 and W is CH(OH).

The compound of formula (III) may be generated in situ by removal of a protecting group from a compound of the formula (Va) 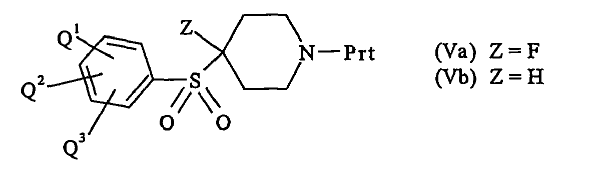 wherein Prt is a protecting group, for example a t-butoxycarbonyl group which can be removed by treatment with trifluoroacetic acid or HCl/ethanol.

The compounds of formula (Va) can be prepared from the corresponding compounds of the formula (Vb) by reaction with a strong base such as nBuLi and a fluorinating agent such as (PhSO₂)₂NF. This reaction will normally take place in a solvent such as tetrahydrofuran at a temperature of -78°C to 25°C.

The compounds (Vb) may be obtained by reaction of a thiol of formula (VI) with a piperidine of formula (VII) and oxidation of the resulting thioether: 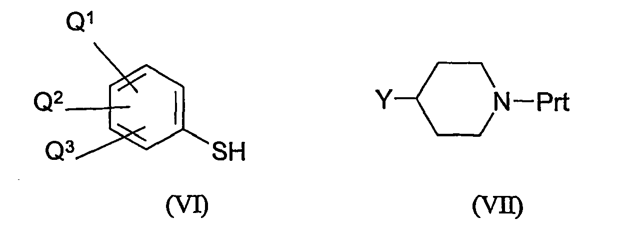 where Y is a leaving group (preferably mesylate or a similar sulphonate ester) and all other variables are as defined previously. Formation of the thioether takes place in refluxing acetonitrile in the presence of a base such as K₂CO₃ and the oxidation may be effected by conventional means (e.g. using m-chloroperoxybenzoic acid or by refluxing with oxone and wet alumina in chloroform). Details of these procedures are provided in WO 00/43362 and *J*. *Med. Chem.,* 2002, **45**, 492-503.

If desired, the aforementioned oxidation may be carried out in two stages via the corresponding sulphoxide intermediate (e.g. by using the oxone method at ambient temperature as the first step). The sulphoxide may be fluorinated in the 4-position of the piperidine ring by treatment with a fluorinating agent such as diethylaminosulfur trifluoride (DAST) (typically in the presence of antimony trifluoride in an inert solvent such as dichloromethane at ambient temperature). Treatment of the resulting compound with an oxidizing agent such as metachloroperbenzoic acid (for example in an inert solvent such as dichloromethane at ambient temperature) then provides compounds (Va).

The compounds of the formula (I) may also be prepared by the fluorination of the corresponding compound which possesses a hydrogen atom instead of the fluorine atom of the compound of formula (I). This may be effected using a fluorinating agent such as (PhSO₂)₂NF and a strong base such as NaHMDS at -78°C to 25°C in an inert solvent such as tetrahydrofuran.

Where they are not commercially available, the starting materials of formula VI and VII may be prepared by procedures analogous to those described in the accompanying Examples, or by standard methods well known from the art.

It will be appreciated that any compound of formula I initially obtained from any of the above processes may, where appropriate, subsequently be elaborated into a further desired compound of formula I using techniques known from the art. For example, a compound of formula I initially obtained wherein Q¹ represents bromo may be converted into the corresponding compound of formula I wherein Q¹ represents cyano by treatment with copper(I) cyanide in the presence of 1-methyl-2-pyrrolidinone (NMP), or with zinc cyanide in the presence of tetrakis(triphenylphosphine)palladium(0). The resulting compound of formula I
wherein Q¹ represents cyano thereby obtained may in turn be converted into the corresponding compound of formula I wherein Q¹ represents carboxamido by heating in mineral acid, e.g. 85% sulphuric acid at 100°C, or by treatment with potassium trimethylsilanolate, typically in tetrahydrofuran at reflux. Alternatively, a compound of formula I initially obtained wherein Q¹ represents bromo may be converted directly into the corresponding compound of formula I wherein Q¹ represents carboxamido by heating under a carbon monoxide atmosphere in the presence of 1,1,1,3,3,3-hexamethyldisilazane, diisopropylamine, palladium(II) acetate and 1,3-bis(diphenylphosphino)propane. Where, for example, Q¹ in the compounds of formula I represents an optionally substituted N-linked hetercyclic moiety, e.g. imidazol-1-yl, pyrazol-1-yl, 1,2,3-triazol-1-yl, 1,2,4-triazol-1-yl, pyrrolidin-1-yl, piperidin-1-yl or azetidin-1-yl, these compounds may be prepared by treating the corresponding compound of formula I wherein Q¹ represents fluoro with the appropriate optionally substituted N-heterocycle, typically with heating in DMSO. Where, for example, Q¹ in the compounds of formula I represents an optionally substituted C-linked five-membered heteroaromatic ring, e.g. 2-methyltetrazol-5-yl or 1-methyl-1,2,4-triazol-5-yl, these compounds may be prepared by reacting the corresponding compound of formula I wherein Q¹ represents bromo with a tributylstannyl derivative of the appropriate heteroaromatic compound, e.g. 2-methyl-5-tributylstannyltetrazole or 1-methyl-5-tributylstannyl-1,2,4-triazole, in the presence of a transition metal catalyst such as tetrakis(triphenylphosphine)palladium(0), typically with heating in a solvent such as *N,N-*dimethylformamide.

Similarly, compounds wherein W is CO may be reduced to provide corresponding compounds wherein W is CH(OH). These in turn may be treated with DAST to provide compounds wherein W is CHF.

Where the above-described processes for the preparation of the compounds of use in the invention give rise to mixtures of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques such as preparative HPLC, or the formation of diastereomeric pairs by salt formation with an optically active acid, such as di-*p*-toluoyl-D-tartaric acid and/or di-*p*-toluoyl-L-tartaric acid, followed by fractional crystallization and regeneration of the free base. The compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in *Protective Groups in Organic Chemistry,* ed. J.F.W. McOmie, Plenum Press, 1973; and T.W. Greene & P.G.M. Wuts, *Protective Groups in Organic Synthesis,* John Wiley & Sons, 1991. The protecting groups may be removed at a convenient subsequent stage using methods known from the art. Compounds were tested for their binding to the 5-HT_{2A} receptor and to other receptors such as 5-HT_{2C} and IKr using the methodology described in Fletcher *et al, J. Med. Chem.,* 2002, **45**, 492-503.

### EXAMPLE 1

### 4-(4-Bromophenylsulfonyl)-1-[2-(2,4-Difluorophenyl)ethyl]-4-fluoropiperidine

To a suspension of wet alumina (10% water, 13 g) in chloroform (60 mL), was added, N-BOC 4-(4-bromophenylthio)piperidine (prepared according to Fletcher, S. R*. et al., J. Med. Chem.* 2002, **45**, 492-503), followed by Oxone^{®} (24 g). The reaction was stirred for 3 h, filtered and chromatographed on silica eluting with 50% EtOAc/isohexane to obtain N-BOC 4-(4-bromophenylsulfinyl)piperidine (3.5 g, 69%): δ_{H} (400 MHz, CDCl₃) 1.44 (9H, s), 1.55-1.8 (4H, m), 2.6-2.75 (3H, m), 4.25 (2H, m), 7.47 (2H, d), 7.67 (2H, d).

N-BOC 4-(4-bromophenylsulfinyl)piperidine (3.17 g, 8.5 mmol) was dissolved in dichloromethane (50 mL) and antimony trichloride (194 mg, 0.85 mmol) was added, followed by diethylaminosulfur trifluoride (2.8 mL, 0.022 mol). The reaction mixture was stirred for 16 h at room temperature, then quenched with cold, saturated sodium bicarbonate solution and the product extracted into ethyl acetate. The organic layer was washed with brine, dried over MgSO₄ and evaporated. The residue was dissolved in dichloromethane (50 mL) and added to a solution of *m*CPBA (50-55%; 7.3 g, 0.021 mol) in dichloromethane (30 mL), which had been previously dried over MgSO₄ and filtered. The reaction mixture was stirred at 25°C for 2 h, then quenched with saturated sodium metabisulfite. The product was extracted into dichloromethane, and the organic layer washed with saturated sodium bicarbonate solution and brine. After drying over MgSO₄, the solvent was removed *in vacuo* and the residue chromatographed on silica, eluting with 10% EtOAc/isohexane to obtain N-BOC 4-(4-bromophenylsulfonyl)-4-fluoropiperidine as a white solid (2.3 g, 70%): δ_{H} (360 MHz, CDCl₃) 1.46 (9H,s) 1.9 (2H, m), 2.02-2.3 (2H, m), 2.9 (2H, m), 4.2 (2H, m), 7.8 (4H, dd).

N-BOC 4-(4-Bromophenylsulfonyl)-4-fluoropiperidine (2.3 g, 5.5 mmol) was dissolved in 6 N HCl (30 mL) and ethanol was added until the cloudy solution became clear (ca. 20 mL). The reaction mixture was heated to 85°C and stirred at that temperature for 16 h. The reaction mixture was cooled, the ethanol evaporated and the acidic solution made basic by the addition of 5 N NaOH. The product was extracted into EtOAc and the organic layer washed with brine and dried over MgSO₄. 4-(4-Bromophenylsulfonyl)-4-fluoropiperidine hydrogen chloride was obtained as a white solid after evaporation of the solvent *in vacuo* (1.4 g, 80%): □_{H} (360 MHz, CDCl₃) 1.84 (2H, dt), 2.04-2.22 (2H, m), 2.82 (2H, dt), 3.09 (2H, dt), 7.75 (4H, m). *m*/*z* (ES⁺) 322,324 (M+1).

4-(4-Bromophenylsulfonyl)-4-fluoropiperidine hydrogen chloride (1.4 g, 4mmol) was dissolved in acetonitrile and potassium carbonate (1.4 g, 0.01mol) was added, followed by 2,4-difluorophenethyl bromide (prepared as described in WO 00/43362, 1.3 g, 6 mmol). The reaction was heated to reflux for 16 h. After cooling, the reaction mixture was partitioned between EtOAc and water and the organic layer washed with brine, dried over MgSO₄ and evaporated. The residue was chromatographed on silica eluting with 30% EtOAc/isohexane to obtain the title compound as a white solid (0.950 g, 51%): δ_{H} (360 MHz, CDCl₃) 1.86 (2H, t), 2.22-2.39 (4H, m), 2.57 (2H, t), 2.76 (2H, t), 2.93 (2H, m), 6.76 (2H, m), 7.13 (1H, m), 7.74 (4H, m). *m*/*z* (ES⁺) 462, 464 (M+1).

### EXAMPLE 2

### 1-12-(2,4-Difluorophenyl)ethyl]-4-fluoro-4-(4-fluorophenylsulfonyl)piperidine Method 1

Sodium bis(trimethylsilyl)amide (1.0 M in THF; 0.3 mL, 0.3 mmol) was added dropwise to a stirred solution of 1-[2-(2,4-difluorophenyl)ethyl]-4-(4-fluorophenylsulfonyl)piperidine (95.8 mg, 0.25 mmol) (prepared according to Fletcher, S. R. *et al., J. Med. Chem.* 2002, **45**, 492-503) in THF (0.5 mL) at -78°C. The solution was warmed to 0°C, stirred for 5 min, then re-cooled to -78°C. A solution of N-fluorobis(phenylsulfonyl)amine (80.8 mg, 0.38 mmol) in THF (0.25 mL) was added and the mixture was allowed to reach ambient temperature, then stirred for 15 min. The reaction was quenched by addition of saturated aqueous NH₄Cl (1 mL), then partitioned between water (20 mL) and EtOAc (20 mL). The organic fraction was dried over Na₂SO₂, filtered and concentrated *in vacuo* and the crude material purified by column chromatography (silica, 30% EtOAc/isohexane) to afford the title compound as a white solid (19 mg): δ_{H} (500 MHz, DMSO) 1.79-1.83 (2H, m), 2.20-2.19 (4H, m), 2.52-2.55 (2H, m), 2.73-2.76 (2H, m), 2.95-2.97 (2H, m), 7.00-7.04 (1H, m), 7.14-7.19 (1H, m), 7.36-7.41 (1H, m), 7.56-7.61 (2H, m), 7.96-7.99 (2H, m). *m*/*z* (ES⁺) 402 (100%, [MH]⁺).

### Method 2

n-Butyllithium (1.6 M in isohexanes; 13 mL, 20.8 mmol) was added dropwise to a stirred solution of N-BOC 4-(4-fluorophenylsulfonyl)piperidine (6 g, 17.4 mmol) in THF (70 mL) at -78°C. After 1 h, a solution of N-fluorobis(phenylsulfonyl)amine (6.04 g, 19 mmol) in THF (17 mL) was added dropwise and the mixture was brought to ambient temperature, stirred for 1 h, then quenched by the addition of water (1 mL) and partitioned between saturated aqueous NH₄Cl (80 mL) and EtOAc (80 mL). The organic fraction was washed with brine (30 mL), dried over Na₂SO₄, filtered and concentrated *in vacuo.* Purification by column chromatography (silica, 20% EtOAc/isohexane) afforded N-BOC 4-fluoro-4-(4-fluorophenylsulfonyl)piperidine as an off-white solid (4.8 g): δ_{H} (360 MHz, CDCl₃) 1.46 (9H, s), 1.80-1.95 (2H, m), 2.05-2.30 (2H, m), 2.90-3.00 (2H, m), 4.12-4.22 (2H, m), 7.28 (2H, t, *J* 8.5 Hz), 7.93-7.96 (2H, m). *m*/*z* (ES⁺) 261 [(M-BOC)H]⁺.

N-BOC 4-fluoro-4-(4-fluorophenylsulfonyl)piperidine (2 g, 5.5 mmol) was suspended in a mixture of EtOH (50 mL) and 6 N HCl (25 mL) and the mixture heated to 80°C until dissolution occurred. The solution was then concentrated *in vacuo* and the resulting residue washed with a 10:1 mixture of Et₂O and EtOAc (50 mL) to afford 4-fluoro-4-(4-fluorophenylsulfonyl)piperidine hydrogen chloride as a white solid.

4-Fluoro-4-(4-fluorophenylsulfonyl)piperidine hydrogen chloride was alkylated with difluorophenethyl bromide as described in Example 1 and recrystallised from 50% EtOAc/isohexane to afford the title compound, analytically consistent with the previous data.

### EXAMPLE 3

### 4-({1-[2-(2,4-Difluorophenyl)ethyl]-4-fluoropiperidin-4-yl}sulfonyl)benzonitrile

### Method 1

4-(4-Bromophenylsulfonyl)-1-[2-(2,4-difluorophenyl)ethyl]-4-fluoropiperidine (0.350 g, 0.758 mmol) was dissolved in anhydrous DMF (10 mL) and zinc cyanide (0.090 g, 0.758 mmol) added. The solution was degassed with nitrogen for 5 minutes and tetrakis(triphenylphosphine) palladium (0) (100 mg) added. The reaction was heated to 85°C for 2 h. Another 100 mg of tetrakis(triphenylphosphine)palladium (0) was added followed by another 100mg after 0.5 h. Another 100 mg of palladium catalyst was then added and the reaction heated at 95°C for 72 h. The reaction mixture was partitioned between EtOAc and water, the organic layer collected and washed with water (x 3) and brine, then dried over MgSO₄. The solvent was removed *in vacuo* and the residue chromatographed on silica eluting with 10-25% EtOAc/isohexane to obtain a solid that was recrystallised from EtOAc/isohexane to give the title compound as a white solid (0.153 g): δ_{H} (400MHz, CDCl₃) 1.87 (2H, m), 2.33 (4H, m), 2.58 (2H, m), 2.76 (2H, m), 2.94 (2H, m), 6.78 (2H, m), 7.13 (1H, m), 7.88 (2H, d), 8.04 (2H, d).

### Method 2

1-[2-(2,4-Difluorophenyl)ethyl]-4-fluoro-4-(4-fluorophenylsulfonyl)piperidine (25 mg, 0.062 mmol), sodium cyanide (20 mg, 0.41 mmol) and DMSO (0.5 mL) were combined and heated to 120°C for 18 h. On cooling, water (5 mL) was added and the resulting precipitate isolated by filtration. The residue was washed with water (10 mL) and dried by a continuous stream of air to afford the title compound (10 mg), analytically consistent with the previous data.

### Method 3

N-BOC 4-(4-bromophenylsulfonyl)piperidine (25 g, 62 mmol) (prepared according to Fletcher, S. R. *et al., J. Med. Chem.* 2002, **45**, 492-503), zinc cyanide (8.73 g, 74 mmol) and tetrakis(triphenylphosphine)palladium (0) (3.58 g, 3.1 mmol) were combined in N-methylpyrrolidine (250 mL) and the mixture heated at 160°C for 30 min. On cooling, the reaction mixture was partitioned between EtOAc (250 mL) and saturated aqueous NaHCO₃ (200 mL). The organic fraction was washed with water (2 x 150 mL) and brine (150 mL), dried over MgSO₄ and concentrated *in vacuo* to approximately 100 mL, at which point precipitation began to occur. Et₂O (500 mL) was added and the resulting precipitate was filtered off, washed with further Et₂O (200 mL) and dried in a vacuum oven to afford N-BOC 4-(piperidin-4-ylsulfonyl)benzonitrile (9.9 g). δ_{H} (500 MHz, DMSO) 1.30-1.40 (2H, m), 1.37 (9H, s), 1.80-1.85 (2H, m), 2.65-79 (2H, m), 3.60-3.68 (1H, m), 3.95-4.05 (2H, m), 8.04 (2H, d, *J* 8.5 Hz), 8.17 (2H, d, *J* 8.5 Hz).

n-Butyllithium (1.6 M in isohexanes; 18.3 mL, 29 mmol) was added to a stirred solution of 2,2,6,6-tetramethylpiperidine (5.4 mL, 32 mmol) in THF (50 mL) at -78°C followed, after 5 min, by a solution of N-BOC 4-(piperidin-4-ylsulfonyl)benzonitrile (9.9 g, 29.3 mmol) in THF (100 mL). The solution was warmed to -10°C and stirred for 90 min prior to addition of a solution of N-fluorobis(phenylsulfonyl)amine (10.1 g, 32 mmol) in THF (40 mL). The reaction mixture was warmed to ambient temperature, stirred for 30 min, then partitioned between EtOAc (150 mL) and saturated aqueous NH₄Cl (150 mL). The organic fraction was dried over MgSO₄, filtered and concentrated *in vacuo* and the crude product purified by column chromatography (silica, 20% EtOAc/isohexane) to afford N-BOC 4-(4-fluoropiperidin-4-ylsulfonyl)benzonitrile (6 g): δ_{H} (500 MHz, DMSO) 1.41 (9H, s), 1.82-1.90 (2H, m), 1.96-1.11 (2H, m), 2.82-2.93 (2H, m), 3.95-4.05 (2H, m), 8.09 (2H, d, *J* 8.2 Hz), 8.22 (2H, d, *J* 8.5 Hz).

N-BOC 4-(4-fluoropiperidin-4-ylsulfonyl)benzonitrile was deprotected and alkylated by the methods described in Examples 2 and 1 respectively to afford the title compound, analytically consistent with the previous data.

### EXAMPLE 4

### 4-({1-[2-(2,4-Difluorophenyl)ethyl]-4-fluoropiperidin-4-yl}sulfonyl)benzamide

Water (0.5 mL), K₂CO₃ (9 mg, 0.065 mmol) and aqueous hydrogen peroxide (*circa* 50%, 0.05 mL, *circa* 0.7 mmol) were added sequentially to a vigorously stirred solution of 4-({1-[2-(2,4-difluorophenyl)ethyl]-4-fluoropiperidin-4-yl}sulfonyl)benzonitrile (53 mg, 0.13 mmol) in DMSO (1 mL). After 30 min, the reaction mixture was partitioned between EtOAc (20 mL) and brine (20 mL) and the organic fraction dried over Na₂SO₄, filtered and concentrated *in vacuo.* Purification by column chromatography (silica, 10% EtOH/EtOAc) afforded the title product as a white solid (8 mg): δ_{H} (500 MHz, DMSO) 1.75-1.85 (2H, m), 2.02-2.20 (4H, m), 2.50-2.55 (2H, m), 2.70-2.78 (2H, m), 2.91-2.99 (2H, m), 6.98-7.02 (1H, m), 7.11-7.18 (1H, m), 7.31-7.41 (1H, m), 7.81 (1H, s), 7.97 (2H, d *J* 8.4 Hz), 8.13 (2H, d *J* 8.3 Hz), 8.25 (1H, s). m/z (ES⁺) 427 [MH]⁺

### EXAMPLE 5

### 4-({4-Fluoro-1-[2-(2-fluorophenyl)ethyl]piperidin-4-yl}sulfonyl)benzamide

A solution of N-BOC 4-(4-fluoropiperidin-4-ylsulfonyl)benzonitrile (1 g, 2.7 mmol) and potassium trimethylsilanolate (346 mg, 2.7 mmol) in toluene (12 mL) was heated to reflux for 2 h. On cooling, the reaction mixture was partitioned between saturated aqueous NH₄Cl (20 mL) and EtOAc (20 mL) and the aqueous portion extracted with further EtOAc (2 x 20 mL). The combined organic fractions were dried over MgSO₄, filtered and concentrated and the residue purified by column chromatography (silica, 30-80% EtOAc/isohexane) to afford N-BOC 4-(4-fluoropiperidin-4-ylsulfonyl)benzamide (350 mg): δ_{H} (500 MHz, DMSO) 1.47 (9H, s), 1.82-1.94 (2H, m), 2.12-2.28 (2H, m), 2.89-2.99 (2H, m), 4.10-4.20 (2H, m), 5.90-6.00 (1H, m), 6.18-6.28 (1H, m), 8.02 (4H, s).

N-BOC 4-(4-fluoropiperidin-4-ylsulfonyl)benzamide was deprotected and alkylated by the methods described in Examples 2 and 1 respectively to afford the title compound as a white solid. δ_{H} (500 MHz, DMSO) 1.78-1.83 (2H, m), 2.05-2.20 (4H, m), 2.53-2.56 (2H, m), 2.74-2.77 (2H, m), 2.96-2.97 (2H, m), 7.10-7.14 (2H, m), 7.22-7.26 (1H, m), 7.30-7.34 (1H, m), 7.70 (1H, s), 7.97 (2H, d *J* 8.3 Hz), 8.12-8.14 (2H, m), 8.25 (1H, s). *m*/*z* (ES⁺) 409 [MH]⁺.

The following compounds (**Examples 6-22**) were prepared using analogous methods to Examples 1-5:
4-Fluoro-4-(4-fluorophenylsulfonyl-1-[2-(4-fluorophenyl)ethyl]piperidine *m*/*z* (ES⁺) 384 [MH]⁺.
4-Fluoro-4-(4-fluorophenylsulfonyl)-1-[2-(2-fluorophenyl)ethylpiperidine *m*/*z* (ES⁺) 384 [MH]⁺.
4-Fluoro-4-(4-fluorophenylsulfonyl)-1-(2-phenylethyl)piperidine *m*/*z* (ES⁺) 366 [MH]⁺.
4-({4-Fluoro-1-[2-(4-fluorophenyl)ethyl]piperidin-4-yl)sulfonyl)benzonitrile *m*/*z* (ES⁺) 391 [MH]⁺.
4-({4-Fluoro-1-[2-(2-fluorophenyl)ethyl]piperidin-4-yl}sulfonyl)benzonitrile *m*/*z* (ES⁺) 391 [MH]⁺.
4-{[4-Fluoro-1-(2-phenylethyl)piperidin-4-yl]sulfonyl}benzonitrile *m*/*z* (ES⁺) 373 [MH]⁺.
4-({4-Fluoro-1-[2-(4-fluorophenyl)ethyl]piperidin-4-yl}sulfonyl)benzamide *m*/*z* (ES⁺)409 [MH]⁺.
4-{[4-Fluoro-1-(2-phenylethyl)piperidin-4-ylsulfonyl}benzamide *m*/*z* (ES⁺)391 [MH]⁺.
1-[2-(2,4-Difluorophenyl)ethyl]-4-fluoro-4-(phenylsulfonyl)piperidine *m*/*z* (ES⁺)384 [MH]⁺.
4-Fluoro-1-(2-(2-fluorophenyl)ethyl]-4-[phenylsulfonyl)piperidine *m*/*z* (ES⁺)366 [MH]⁺.
4-Fluoro-1-(2-(4-fluorophenyl)ethyl]-4-(phenylsulfonyl)piperidine *m*/*z* (ES⁺)366 [MH]⁺.
4-Fluoro-1-(2-phenylethyl)-4-(phenylsulfonyl)piperidine *m*/*z* (ES⁺) 348 [MH]⁺.
1-[2-(2,4-Difluorophenyl)ethyl]-4-fluoro-4-(2-fluorophenylsulfonyl)piperidine *m*/*z* (ES⁺) 402 [MH]⁺.
1-[2-(2,4-Difluorophenyl)ethyl]-4-fluoro-4-(3-fluorophenylsulfonyl)piperidine *m*/*z* (ES⁺) 402 [MH]⁺.
4-(4-Chlorophenylsulfonyl)-1-[2-(2,4-difluorophenyl)ethyl]-4-fluoropiperidine *m*/*z* (ES⁺) 419 [MH]⁺.
4-({4-Fluoro-1-[(6-fluoro-1,2-benzisothiazol-3-yl)methyl]piperidin-4-yl} sulfonyl)benzonitrile
   *m*/*z* (ES⁺) 434 [MH]⁺.
6-Fluoro-3-({4-fluoro-4-[(4-fluorophenyl)sulfonyl]piperidin-1-yl}methyl)-1,2-benzisothiazole
   *m*/*z* (ES⁺) 427 [MH]⁺.

### EXAMPLE 23

### 4-[4-({1-[2-(2,4-Difluorophenyl)ethyl]-4-fluoropiperidin-4-yl}sulfonyl)phenyl]morpholine

4-(4-Bromophenylsulfonyl)-1-[2-(2,4-difluorophenyl)ethyl]-4-fluoropiperidine (25 mg, 0.054 mmol) was dissolved in anhydrous toluene (1.5 mL). Morpholine (0.01 mL, 0.108 mmol) and *rac*-2,2'-bis(diphenylphosphino)-1,1'-binaphthyl (2.7 mg; 5 mol%) was added. The solution was degassed with nitrogen for 5 minutes and palladium acetate (1.1 mg; 2 mol%) and cesium carbonate (26 mg, 0.082 mmol) added. The reaction was heated to 90°C for 16 h. The reaction mixture was partitioned between dichloromethane and water and the organic layer was collected and washed with water and brine, then dried over Na₂SO₄. The solvent was removed *in vacuo* and the residue chromatographed on silica eluting with 70% EtOAc/isohexane to give the title compound as a white solid (16 mg): δ_{H} (400 MHz, DMSO) 1.75 (2H, m),1.97 (2H, m), 2.09 (2H, m), 2.71 (2H, m), 2.92 (2H, m), 3.34 (4H, m), 3.72 (4H, m), 6.97 (1H, m), 7.08 (3H, m), 7.35 (1H, m), 7.61 (2H, m). *m*/z (ES⁺) 469 [MH]⁺.

### EXAMPLE 24

### 1-[2-(2,4-Difluorophenyl)ethyl]-4-fluoro-4-[4-(pyrrolidin-1-yl)phenylsulfonyl]piperidine

4-(4-Fluorophenylsulphonyl}-1-[2-(2,4-difluorophenyl)ethyl]-4-fluoropiperidine (100 mg, 2.44 mmol) was dissolved in DMSO (5 ml). Pyrrolidine (0.61 mL, 7.32 mmol) was added and the resultant solution heated to 150°C in a microwave reactor for 10 minutes. On cooling, the reaction mixture was partitioned between EtOAc (30 mL) and aqueous saturated NaHCO₃ (30 mL). The organic layer was washed with water, dried over Na₂SO₄, filtered and concentrated *in vacuo* to give the title compound as a white solid requiring no purification (102 mg): δ_{H} (400 MHz, DMSO) 1.75 (2H, m) 2.0 (4H, m) 2.1 (2H, m) 2.5 (2H, m) 2.7 (2H, m) 2.95 (2 H, m) 3.35 (4H, m) 6.7 (2H, d) 7.0 (1H, m) 7.15 (1H, m) 7.4 (1H, m) 7.6 (2H, d). *m*/*z* (ES⁺) 453 [MH]⁺.

The following compounds (**Examples 25-31**) were prepared using analogous methods to Examples 23-24:
1-[2-(2,4-Difluorophenyl)ethyl]-4-fluoro-4-[4-(piperidin-1-yl)phenylsulfonyl]piperidine
   *m*/*z* (ES⁺) 467 [MH]⁺.
1-[2-(2,4-Difluorophenyl)ethyl]-4-fluoro-4-{4-[4-(trifluoromethyl)piperidin-1-yl]phenylsulfonyl}piperidine
   *m*/*z* (ES⁺) 535 [MH]⁺.
4-[4-(Azetidin-1-yl)phenylsulfonyl]-1-[2-(2,4-difluorophenyl)ethyl]-4-fluoropiperidine
   *m*/*z* (ES⁺) 439 [MH]⁺.
1-[2-(2,4-Difluorophenyl)ethyl]-4-[4-(4,4-difluoropiperidin-1-yl)phenylsulfonyl]-4-fluoropiperidine
   *m*/*z* (ES⁺) 503 [MH]⁺.
1-[2-(2,4-Difluorophenyl)ethyl]-4-fluoro-4-[4-([1,2,3]triazol-1-yl)phenylsulfonyl]piperidine
   *m*/*z* (ES⁺) 451 [MH]⁺.
1-[2-(2,4-Difluorophenyl)ethyl]-4-fluoro-4-[4-(pyrrol-1-yl)phenylsulfonyl]piperidine *m*/*z* (ES⁺) 449 [MH]⁺.
1-[2-(2,4-Difluorophenyl)ethyl]-4-fluoro-4-[4-(pyrazol-1-yl)phenylsulfonyl]piperidine
   *m*/*z* (ES⁺) 450 [MH]⁺.

The following compounds **(Examples 32-35)** were prepared from 4-(3,4-difluorophenylsulphonyl)-1-[2-(2,4-difluorophenyl)ethyl]-4-fluoropiperidine by an analogous method to Example 24.
1-[2-(2,4-Difluorophenyl)ethyl]-4-fluoro-4-{[3-fluoro-4-(pyrazol-1-yl)phenyl]sulfonyl]piperidine
   m/z (ES⁺) 468 [MH⁺].
1-[2-(2,4-Difluorophenyl)ethyl]-4-fluoro-4-{[3-fluoro-4-(imidazol-1-yl)phenyl] sulfonyl}piperidine
   *m*/*z* (ES⁺)468 [MH]⁺.
1-[2-(2,4-Difluorophenyl)ethyl]-4-fluoro-4-{[3-fluoro-4-(1,2, 3-triazol-1-yl)phenyl]sulfonyl}piperidine
   *m*/*z* (ES⁺) 469 [MH]⁺.
1-r2-(2,4-Difluorophenyl)ethyll-4-fluoro-4-f {[3-fluoro-4-(1,2,4-triazol-1-yl)phenyl]sulfonyl}piperidine
   *m*/*z* (ES⁺) 469 [MH]⁺.

### EXAMPLE 36

### 1-[2-(2,4-Difluorophenyl)ethyl]-4-fluoro-4-[4-(1,3-oxazol-2-yl)phenylsulfonyl]piperidine

4-(4-Bromophenylsulphonyl)-1-[2-(2,4-difluorophenyl)ethyl]-4-fluoropiperidine (50 mg, 0.108 mmol) and 2-(tributylstannyl)oxazole (0.04 mL, 0.119 mmol) were dissolved in anhydrous THF (0.25 mL) and the solution was degassed with nitrogen for 5 minutes prior to addition of bis(triphenylphosphine)palladium(II) chloride (3.8 mg, 5 mol%). The reaction mixture was heated at 80°C for 72 h, then partitioned between DCM (20 mL) and water (20 mL) and the organic layer dried over Na₂SO₄, filtered and concentrated *in vacuo.* The resultant residue was diluted with DCM (0.5 mL) and passed through a SCX cartridge and the absorbed material washed with further DCM (10 mL). The product eluted on introduction of ammonia in MeOH (2M, 10 mL). The fractions were combined, the volatiles evaporated and the residue purified by column chromatography (silica, 65% EtOAc/isohexane) to give the title compound as a white solid (28 mg): δ_{H} (400 MHz, DMSO) 1.8 (2H, m) 2.1 (4H, m) 2.55 (2H, m) 2.7 (2H,m) 2.95 (2H, m) 7.0 (1H, m) 7.15 (1H, m) 7.4 (1H, m) 7.55 (1H, s) 8.05 (2H, d) 8.29 (2H, d) 8.35 (1H, s). *m*/*z* (ES⁺) 451 [MH]⁺.

The following compounds **(Examples 37-40)** were prepared using analogous methods to Example 36:
1-[2-(2,4-Difluorophenyl)ethyl]-4-fluoro-4-[4-(1-methylpyrazol-5-yl)phenylsulfonyllpiperidine
   *m*/*z* (ES) 464 [MH]⁺.
1-[2-(2,4-Difluorophenyl)ethyl]-4-fluoro-4-f 4-(1-methylimidazol-2-yl-)phenylsulfonyl]piperidine
   *m*/*z* (ES⁺) 464 [MH]⁺.
1-[2-(2,4-Difluorophenyl)ethyl]-4-fluoro-4-[4-(2-methyltetrazol-5-yl)phenylsulfonyllpiperidine
   *m*/*z* (ES⁺) 466 [MH]⁺.
1-[2-(2,4-Difluorophenyl)ethyl]-4-fluoro-4-[4-(1,3-thiazol-2-yl)phenylsulfonyl]piperidine
   *m*/*z* (ES⁺) 467 [MH]⁺.

### EXAMPLE 41

### 4-[(2-Bromo-4-fluorophenyl)sulfonyl]-1-[2-(2,4-difluorophenyl)ethyl]-4-fluoropiperidine

n-Butyllithium (1.6 N in isohexanes; 1.0 mL, 1.6 mmol) was added dropwise to a stirred solution of N-BOC 4-fluoro-4-(4-fluorophenylsulfonyl)piperidine (500 mg; 1.4 mmol) in diethyl ether (16 mL) at -78C. After 1 h, 1,2-dibromotetrafluoroethane (0.54 g, 2.1 mmol) was added briskly and the mixture stirred for 5 minutes prior to warming to 0°C at which temperature it was stirred for a further 1.5 h. Saturated aqueous NH₄Cl (1 mL) was added and the mixture partitioned between water (20 mL) and EtOAc (20 mL). The organic portion was dried over Na₂SO₄, filtered and concentrated *in vacuo* to afford crude product which was deprotected and alkylated as described in Examples 2 and 1 respectively. The final material was washed with a 50% Et₂O/isohexane to afford the title compound as a white solid (117 mg): δ_{H} (400 MHz, DMSO) 1.75-1.90 (2H, m), 2.04-2.22 (4H, m), 2.50-2.55 (2H, m), 2.70-2.75 (2H, m), 2.95-3.00 (2H, m), 6.98-7.02 (1H, m), 7.12-7.18 (1H, m), 7.35-7.40 (1H, m), 7.58-7.62 (1H, m), 7.98-8.00 (1H, m), 8.10-8.13 (1H, m). *m*/*z* (ES⁺) 482, 480 [MH]⁺. The material also contained a 3.6% impurity of 1-[2-(2,4-difluorophenyl)ethyl]-4-fluoro-4-(4-fluorophenylsulfonyl)piperidine, consistent with the data presented in Example 2.

### EXAMPLE 42

### (RS)-1-[2-(2,4-Difluorophenyl)-2-fluoroethyl)-4-fluoro-4-(phenylsulfonyl)piperidine

4-Fluoro-4-(phenylsulfonyl)piperidine hydrogen chloride (300 mg, mmol) was treated with saturated aqueous sodium bicarbonate and the free base extracted into EtOAc. The organic layer was dried over MgSO₄ and the solvent removed *in vacuo* to yield 4-fluoro-4-(phenylsulfonyl)piperidine (187 mg) as a white solid.

2-(2, 4-Difluorophenyl)oxirane (0.17 ml, 1.08 mmol) was added to a solution of 4-fluoro-4-(phenylsulfonyl)piperidine (187 mg, 0.9 mmol) in DMSO (5 ml) and the resultant solution heated to 105°C for 10 hours. On cooling, the reaction solution was poured into a brine solution (10ml) and extracted into EtOAc. The organic layer was washed with water (3 x 10 ml), dried over Na₂SO₄, filtered and concentrated. The resultant residue was purified by column chromatography (silica, 30% EtOAc/isohexane) to give (*RS*)-1-(2,4-difluorophenyl)-2-[4-fluoro-4-(phenylsulfonyl)piperidin-1-yl]ethanol as a white solid (108 mg): δ_{H} (400 MHz, DMSO) 1.75 (2H, m) 2.05 (2H, m) 2.2 (2H, m) 2.5 (2H,m) 2.95 (2 H, m) 4.95 (1H, m) 5.39 (1H, d) 7.05 (1H, m) 7.15 (1H, m) 7.5 (1H, m) 7.72 (2H, m) 7.85 (3H, m). *mlz* (ES⁺) 399 [MH]⁺.

To a cooled (-78°C) solution of (*RS*)-1-(2,4-difluorophenyl)-2-[4-fluoro-4-(phenylsulfonyl)piperidin-1-yl]ethanol (40 mg, 0.10 mmol) in anhydrous DCM (0.5 ml) under a nitrogen atmosphere was added (diethylamino)sulfur trifluoride (0.02 ml, 0.15 mmol). The solution was stirred for 10 minutes at -78 °C, then allowed to warm to room temperature and left to stir for 2 h. The reaction was quenched with saturated aqueous sodium bicarbonate solution (20 ml) and extracted into EtOAc (3 x 20 ml). The combined organic fractions were dried over Na₂SO₄, filtered and concentrated *in vacuo.* The resultant residue was purified by column chromatography (silica, 25% EtOAc/isohexane) to give the title compound as a white solid (29 mg): δ_{H} (400 MHz, DMSO) 1.8 (2H, m) 2.1 (2H, m) 2.2 (2H, m) 2.7 (2H,m) 2.95 (3 H, m) 5.8-5.9 (1H, ddd) 7.15 (1H, m) 7.25 (1H, m) 7.55 (1H, m) 7.72 (2H, m) 7.85 (3H, m). *m*/*z* (ES⁺) 402 [MH]⁺.

### EXAMPLE 43

### 2-[4-Fluoro-4-(4-fluorophenylsulfonyl)piperidin-1-yl]-1-(4-fluorophenyl)ethanone

4-Fluoro-2'-bromoacetophenone (55 mg, 0.25 mmol) was added to a mixture of 4-fluoro-4-(4-fluorophenylsulfonyl)piperidine hydrogen chloride (60 mg, 0.23 mmol) and triethylamine (0.064 mL, 0.50 mmol) in MeCN (1.2 mL). The solution was stirred at ambient temperature for 4 h, then partitioned between water (20 mL) and EtOAc (20 mL). The organic portion was dried over Na₂SO₄, filtered and concentrated *in vacuo* affording crude material, which was purified by column chromatography (silica, 50% EtOAc/isohexane) to yield the title compound as a white solid (30 mg): δ_{H} (500 MHz, DMSO) 1.95 (2H, t, *J* 11.6), 2.20-2.35 (2H, m), 3.08 (2 H, d, *J* 6.5), 4.06 (2H, s), 7.48 (2H, t, *J* 8.9), 7.71 (2H, t, *J* 8.8), 8.11 (2H, dd, *J* 5.2 and 8.7), 8.20 (2H, dd, *J* 5.6 and 8.9). *m*/*z* (ES⁺) 398 [MH]⁺.

### EXAMPLE 44

### (RS)-4-Fluoro-1-[2-fluoro-2-(4-fluorophenyl)ethyl]-4-(4-fluorophenylsulfonyl)piperidine

Sodium borohydride (20 mg, 0.5 mmol) was added to a suspension of 2-[4-fluoro-4-(4-fluorophenylsulfonyl)piperidin-1-yl]-1-(4-fluorophenyl)ethanone (40 mg, 0.1 mmol) in EtOH (5 mL) and the mixture stirred at ambient tempterature for 1 h. The resulting solution was concentrated *in vacuo* and the residue partitioned between water (20 mL) and EtOAc (20 mL). The organic portion was dried over Na₂SO₄, filtered and concentrated to afford (*RS)*-2-[4-fluoro-4-(4-fluorophenylsulfonyl]piperidin-1-yl]-1-(4-fluorophenyl)ethanol as a white solid: *m*/*z* (ES⁺) 418 [MH]⁺.

(*RS)*-2-[4-Fluoro-4-(4-fluorophenylsulfonyl]piperidin-1-yl]-1-(4-fluorophenyl)ethanol was converted to the title compound as described in Example 42: δ_{H} (500 MHz, DMSO) 1.78-1.82 (2H, m), 2.05-2.20 (2H, m), 2.25-2.32 (2H, m), 2.63-2.72 (1H, m), 2.88-3.04 (3H, m), 5.67-5.79 (1H, m), 7.21-7.25 (2H, m), 7.43-7.48 (2H, m), 7.56-7.60 (2H, m), 7.96-7.99 (2H, m). *m*/*z* (ES⁺) 402 [MH]⁺.

The following compounds (**Examples 45-48**) were prepared using analogous methods to Examples 42-44:
4-({4-Fluoro-1-[2-(4-fluorophenyl)-2-oxoethyl]piperidin-4-yl}sulfonyl)benzonitrile
   *m*/*z* (ES⁺) 405 [MH]⁺.
1-(4-Fluorophenyl)-2-[4-fluoro-4-(phenylsulfonyl)piperidin-1-yl]ethanone
   *m*/*z* (ES⁺) 380 [MH]⁺.
(*RS*)-4-({1-[2-(2,4-Difluorolphenyl)-2-fluoroethyl]-4-fluoropiperidin-4-yl} sulfonyl)benzamide
   *m*/*z* (ES⁺) 445 [MH]⁺.
(*RS*)-4-({4-Fluoro-1-[2-fluoro-2-(4-fluorophenyl)ethyl]piperidin-4-yl} sulfonyl)benzamide
   *m*/*z* (ES⁺) 427 [MH]⁺.

### EXAMPLE 49

### (RS)-1-[2-(2,4-Difluorophenyl)-1-methylethyl]-4-fluoro-4-(4-fluorophenylsulfonyl)piperidine

Titanium tetraisopropoxide (0.29 mL, 1.4 mmol) was added to a solution of 4-fluoro-4-(4-fluorophenylsulfonyl)piperidine (179 mg, 0.68 mmol) and 2,4-difluorophenylacetone (116 mg, 0.68 mmol) in EtOH (3.4 mL) and the mixture stirred at ambient temperature for 16 h. Sodium borohydride was added (41 mg, 1.0 mmol) and stirring continued for 2 h. Water (20 mL) and EtOAc (20 mL) were added and the bi-phasic mixture was filtered through Hyflo^{®}. The filtrate was partitioned and the organic portion dried over Na₂SO₄, filtered and concentrated *in vacuo.* Purification by column chromatography (silica, 50% EtOAc/isohexane) afforded semi-pure material which was further purified by treatment with a 1 N solution of HCl in Et₂O, followed by washing of the solid residue with further Et₂O, yielding the title compound as the hydrogen chloride salt: δ_{H} (500 MHz, DMSO)1.09-1.19 (3H, m), 2.14-2.21 (2H, m), 2.53-2.60 (1H, m), 2.68-2.85 (3H, m), 3.13-3.56 (5H, m). 7.08-7.14 (1H, m), 7.22-7.28 (1H, m), 7.41-7.48 (1H, m), 7.60-7.67 (2H, m), 8.00-8.07 (2H, m), 11.10-11.20 (br s). *m*/*z* (ES⁺) 416 [MH]⁺.

## Claims

1. A compound of formula I: 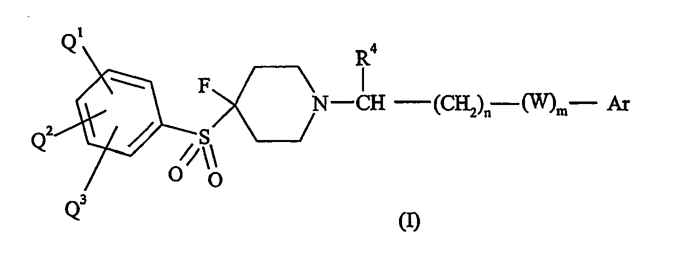 or a pharmaceutically acceptable salt thereof wherein:
Ar is phenyl, benzisothiazol-3-yl or benzthiophen-3-yl, each of which bears substituent groups R¹, R² and R³;
R¹ is hydrogen, fluorine, chlorine, bromine, C₁₋₆ alkyl, C₃₋₆ cycloalkyl, C₂₋₆ alkenyl, C₂₋₆ alkynyl, C₁₋₆ alkoxy, C₂₋₆ alkenyloxy, C₂₋₆ alkynyloxy, or C₁₋₆ alkyl substituted by up to 5-fluorine atoms;
R² is hydrogen, fluorine, chlorine, C₁₋₄ alkyl, C₁₋₄ alkoxy, C₁₋₄ alkyl substituted by up to 5 fluorine atoms or C₁₋₄ alkoxy substituted by up to 5 fluorine atoms;
R³ is hydrogen, fluorine, chlorine, methyl, methoxy, trifluoromethyl, difluoromethyl, trifluoromethoxy or difluoromethoxy;
Q¹ is hydrogen; fluorine; chlorine; bromine; C₁₋₆ alkyl; C₃₋₆ cycloalkyl; C₂₋₆ alkenyl; C₂₋₆ alkynyl; C₁₋₆ alkoxy; C₂₋₆ alkenyloxy; C₂₋₆ alkynyloxy; C₁₋₆ alkyl substituted by up to 5-fluorine atoms; nitrile; COQ⁴ or CO₂Q⁴ where Q⁴ is hydrogen or C₁₋₆ alkyl; NQ⁵Q⁶, CONQ⁵Q⁶ or SO₂NQ⁵Q⁶ where Q⁵ is hydrogen or C₁₋₆alkyl and Q⁶ is hydrogen or C₁₋₆ alkyl or Q⁵ and Q⁶ are joined to form either a 4-7 membered heterocyclic ring which may also contain one oxygen or one further nitrogen ring atom, which heterocyclic ring may optionally be substituted by up to 3 fluorine atoms or by CF₃, methyl, ethyl or hydroxyl; hydroxyl; nitro; SOQ⁷ or SO₂Q⁷ where Q⁷ is C₁₋₄ alkyl; NQ⁸COQ⁹, NQ⁸CO₂Q⁹ or NQ⁸SO₂Q⁹ where Q⁸ is hydrogen or C₁₋₄alkyl and Q⁹ is hydrogen or C₁₋₄alkyl or is joined to Q⁸ to form a 5-7 membered ring; a heteroaromatic ring of 5 ring atoms 1, 2, 3 or 4 of which may be nitrogen atoms or 1 or 2 of which are nitrogen atoms and 1 of which is an oxygen or sulfur atom or 1 of which is an oxygen or sulfur atom, which heteroaromatic ring optionally being substituted by methyl, ethyl or hydroxyl; or a heteroaromatic ring of 6 ring atoms containing 1 or 2 nitrogen ring atoms or a phenyl group either of which is optionally substituted by 1 or 2 fluorine or chlorine atoms or C₁₋₄alkyl, C₁₋₄alkoxy or trifluoromethyl groups;
Q² is hydrogen, fluorine, chlorine, nitrile, hydroxy, C₁₋₄ alkyl, C₁₋₄ alkoxy, C₁₋₄ alkyl substituted by up to 5 fluorine atoms, or C₁₋₄ alkoxy substituted by up to 5 fluorine atoms;
Q³ is hydrogen, fluorine, chlorine, methyl, methoxy, trifluoromethyl, difluoromethyl, trifluoromethoxy or difluoromethoxy;
or Q² and Q³ are joined to form the residue of a 5, 6 or 7 membered carbocyclic ring;
R⁴ is H or C₁₋₄ alkyl,
m is 0 or 1;
n is 0, 1 or 2; and
W is CH₂, CHF, CH(OH) or CO.

2. A compound according to claim 1 wherein Ar represents benzisothiazol-3-yl or benzthiophen-3-yl, each bearing substituent groups R¹, R² and R³, and m and n are both 0.

3. A compound according to claim 1 wherein Ar represents phenyl bearing substituent groups R¹, R² and R³, m is 1 and n is 0.

4. A compound according to claim 1 of formula IIA: 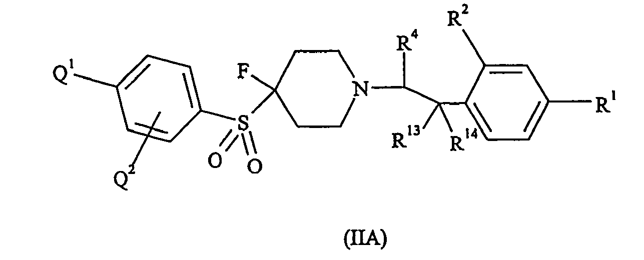 or a pharmaceutically acceptable salt thereof
wherein R¹³ represents H and R¹⁴ represents H, F or OH, or R¹³ and R¹⁴ together represent keto;
and Q¹, Q², R¹, R² and R⁴ are as defined in claim 1.

5. A compound according to any previous claim wherein Q¹ is selected from H, F, Cl, Br, CN, carboxamide, 5-membered heteroaryl and NQ⁵Q⁶ where Q⁵ and Q⁶ complete a heterocyclic ring;
Q² is H, F or Cl;
Q³ is H or F;
R¹ is H, F, methyl or CF₃;
R² is H, F, methyl or CF₃; and
R³ is H.

6. A pharmaceutical composition comprising a compound according to claim 1 and a pharmaceutically acceptable carrier.

7. A compound according to claim 1 for use in a method of treatment of the human body.

8. The use of a compound according to claim 1 for the manufacture of a medicament for treating or preventing a condition mediated by 5-HT_{2A} receptor activity.

## Patentansprüche

1. Eine Verbindung der Formel 1: 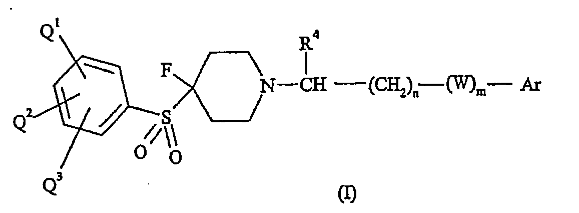 oder ein pharmazeutisch annehmbares Salz davon, wobei:
Ar Phenyl, Benzisothiazol-3-yl oder Benzthiophen-3-yl ist, wobei jedes davon Substituentengruppen R¹, R² und R³ trägt,
R¹ Wasserstoff, Fluor, Chlor, Brom, C₁₋₆-Alkyl, C₃₋₆-Cycloalkyl, C₂₋₆-Alkenyl, C₂₋₆-Alkinyl, C₁₋₆-Alkoxy, C₂₋₆-Alkenyloxy, C₂₋₆-Alkinyloxy oder durch bis zu 5 Fluoratome substituiertes C₁₋₆-Alkyl ist,
R² Wasserstoff, Fluor, Chlor, C₁₋₄-Alkyl, C₁₋₄-Alkoxy, durch bis zu 5 Fluoratome substituiertes C₁₋₄-Alkyl oder durch bis zu 5 Fluoratome substituiertes C₁₋₄-Alkoxy ist,
R³ Wasserstoff, Fluor, Chlor, Methyl, Methoxy, Trifluormethyl, Difluormethyl, Trifluormethoxy oder Difluormethoxy ist,
Q¹ ist Wasserstoff; Fluor; Chlor; Brom; C₁₋₆-Alkyl; C₃₋₆-Cycloalky; C₂₋₆-Alkenyl; C₂₋₆-Alkinyl; C₁₋₆-Alkoxy; C₂₋₆-Alkenyloxy; C₂₋₆-Alkinyloxy; C₁₋₆-Alkyl, substituiert durch bis zu 5 Fluoratome; Nitril; COQ⁴ oder CO₂Q⁴, wobei Q⁴ Wasserstoff oder C₁₋₆-Alkyl ist; NQ⁵Q⁶, CONQ⁵Q⁶ oder SO₂NQ⁵Q⁶, wobei Q⁵ Wasserstoff oder C₁₋₆-Alkyl ist und Q⁶ Wasserstoff oder C₁₋₆-Alkyl ist oder Q⁵ und Q⁶ verbunden sind, um einen 4-7-gliedrigen heterocyclischen Ring zu bilden, der auch ein Sauerstoff- oder ein weiteres Stickstoff-Ringatom enthalten kann, wobei der heterocyclische Ring gegebenenfalls substituiert sein kann durch bis zu 3 Fluoratome oder durch CF₃, Methyl, Ethyl oder Hydroxyl; Hydroxyl; Nitro; SOQ⁷ oder SO₂Q⁷, wobei Q⁷ C₁₋₄-Alkyl ist; NQ⁸COQ⁹, NQ⁸CO₂Q⁹ oder NQ⁸SO₂Q⁹, wobei Q⁸ Wasserstoff oder C₁₋₄-Alkyl ist und Q⁹ Wasserstoff oder C₁₋₄-Alkyl ist oder mit Q⁸ verbunden ist, um einen 5-7-gliedrigen Ring zu bilden; ein heteroaromatischer Ring aus 5 Ringatomen, wobei 1, 2, 3 oder 4 davon Stickstoffatome sein können oder 1 oder 2 davon Stickstoffatome sind und 1 davon ein Sauerstoff- oder Schwefelatom ist oder 1 davon ein Sauerstoff- oder Schwefelatom ist, wobei der iieieroaromaüsche Ring gegebenenfalls substituiert ist durch Methyl, Ethyl oder Hydroxyl; oder ein heteroaromatischer Ring aus 6 Ringatomen, der 1 oder 2 Stickstoff-Ringatome enthält, oder eine Phenylgruppe ist, wobei jedes davon gegebenenfalls substituiert ist durch 1 oder 2 Fluor- oder Chloratome oder C₁₋₄-Alkyl-, C₁₋₄-Alkoxy- oder Trifluormethylgruppen,
Q² Wasserstoff, Fluor, Chlor, Nitril, Hydroxy, C₁₋₄-Alkyl, C₁₋₄-Alkoxy, durch bis zu 5 Fluoratome substituiertes C₁₋₄-Alkyl oder durch bis zu 5 Fluoratome substituiertes C₁₋₄-Alkoxy ist,
Q³ Wasserstoff, Fluor, Chlor, Methyl, Methoxy, Trifluormethyl, Difluormethyl, Trifluormethoxy oder Difluormethoxy ist
oder Q² und Q³ verbunden sind, um den Rest eines 5-, 6- oder 7-gliedrigen carbocyclischen Rings zu bilden,
R⁴ H oder C₁₋₄-Alkyl ist,
m 0 oder 1 ist,
n 0, 1 oder 2 ist und
W CH₂, CHF, CH(OH) oder CO ist.

2. Eine Verbindung gemäß Anspruch 1, wobei Ar Benzisothiazol-3-yl oder Benzthiophen-3-yl bedeutet, wobei jedes Substituentengruppen R¹, R² und R³ trägt, und m und n beide 0 sind.

3. Eine Verbindung gemäß Anspruch 1, wobei Ar Phenyl bedeutet, welches Substituentengruppen R¹, R² und R³ trägt, m 1 ist und n 0 ist.

4. Eine Verbindung gemäß Anspruch 1 der Formel IIA: 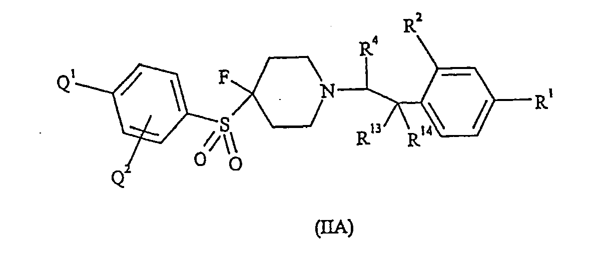 oder ein pharmazeutisch annehmbares Salz davon,
wobei R¹³ H bedeutet und R¹⁴ H, F oder OH bedeutet, oder R¹³ und R¹⁴ zusammen Keto bedeuten,
und Q¹, Q², R¹, R² und R⁴ wie in Anspruch 1 definiert sind.

5. Eine Verbindung gemäß irgendeinem vorherigen Anspruch, wobei Q¹ ausgewählt ist aus H, F, Cl, Br, CN, Carboxamid, 5-gliedrigem Heteroaryl und NQ⁵Q⁶,
wobei Q⁵ und Q⁶ einen heterocyclischen Ring vervollständigen,
Q² H, F oder Cl ist,
Q³ H oder F ist,
R¹ H, F, Methyl oder CF₃ ist,
R² H, F, Methyl oder CF₃ ist und
R³ H ist.

6. Eine pharmazeutische Zusammensetzung, die eine Verbindung gemäß Anspruch 1 und einen pharmazeutisch annehmbaren Träger enthält.

7. Eine Verbindung gemäß Anspruch 1 zur Verwendung bei einem Verfahren zur Behandlung des menschlichen Körpers.

8. Die Verwendung einer Verbindung gemäß Anspruch 1 zur Herstellung eines Medikaments zur Behandlung oder Prävention eines durch 5-HT_{2A}-Rezeptoraktivität vermittelten Zustandes.

## Revendications

1. Composé de formule I: 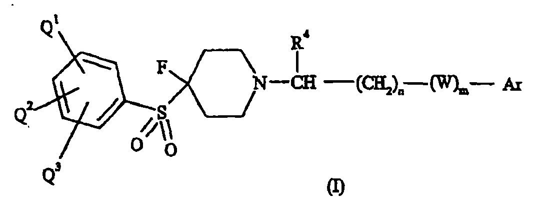 ou un sel pharmaceutiquement acceptable de ce composé, formule dans laquelle :
Ar est un groupe phényle, benzisothiazol-3-yle ou benzothiophén-3-yle, chacun d'eux portant des groupes substituants R¹, R² et R³;
R¹ est un atome d'hydrogène, de fluor, de chlore, de brome, ou un groupe alkyle en C₁₋₆, cycloalkyle en C₃₋₆, alcényle en C₂₋₆, alcynyle en C₂₋₆, alcoxy en C₁₋₆, alcényloxy en C₂₋₆, alcynyloxy en C₂₋₆ ou alkyle en C₁₋₆ substitué par jusqu'à 5 atomes de fluor ;
R² est un atome d'hydrogène, de fluor, de chlore, ou un groupe alkyle en C₁₋₄, alcoxy en C₁₋₄, alkyle en C₁₋₄ substitué par jusqu'à 5 atomes de fluor ou alcoxy en C₁₋₄ substitué par jusqu'à 5 atomes de fluor ;
R³ est un atome d'hydrogène, de fluor, de chlore, ou un groupe méthyle, méthoxy, trifluorométhyle, difluorométhyle, trifluorométhoxy ou difluorométhoxy ;
Q¹ est un atome d'hydrogène; de fluor ; de chlore ; de brome ; ou un groupe alkyle en C₁₋₆; cycloalkyle en C₃₋₆; alcényle en C₂₋₆; alcynyle en C₂₋₆; alcoxy en C₁₋₆; alcényloxy en C₂₋₆; alcynyloxy en C₂₋₆; alkyle en C₁₋₆ substitué par jusqu'à 5 atomes de fluor ; nitrile ; COQ⁴ ou CO₂Q⁴ où Q⁴ est un atome d'hydrogène ou un groupe alkyle en C₁₋₆; NQ⁵Q⁶, CONQ⁵Q⁶ ou SO₂NQ⁵Q⁶ où Q⁵ est un atome d'hydrogène ou un groupe alkyle en C₁₋₆ et Q⁶ est un atome d'hydrogène ou un groupe alkyle en C₁₋₆, ou bien Q⁵ et Q⁶ sont reliés pour former soit un cycle hétérocyclique à 4-7 chaînons qui peut aussi contenir un atome d'oxygène ou un autre atome d'azote dans le cycle, ce cycle hétérocyclique pouvant être éventuellement substitué par jusqu'à 3 atomes de fluor ou par un groupe CF₃, méthyle, éthyle ou hydroxyle ; hydroxyle ; nitro ; SOQ⁷ ou SO₂Q⁷, où Q⁷ est un groupe alkyle en C₁₋₄; NQ⁸COQ⁹, NQ⁸CO₂Q⁹ ou NQ⁸SO₂Q⁹, où Q⁸ est un atome d'hydrogène ou un groupe alkyle en C₁₋₄ et Q⁹ est un atome d'hydrogène ou un groupe alkyle en C₁₋₄ ou bien est relié à Q⁸ pour former un cycle à 5-7 chaînons ; un cycle hétéroaromatique de 5 atomes dans le cycle, dont 1, 2, 3 ou 4 peuvent être des atomes d'azote ou dont 1 ou 2 sont des atomes d'azote et 1 est un atome d'oxygène ou de soufre ou dont 1 est un atome d'oxygène ou de soufre, ce cycle hétéroaromatique étant éventuellement substitué par un groupe méthyle, éthyle ou hydroxyle ; ou un cycle hétéroaromatique de 6 atomes dans le cycle contenant 1 ou 2 atomes d'azote dans le cycle ou un groupe phényle, l'un ou l'autre étant éventuellement substitué par 1 ou 2 atomes de fluor ou de chlore ou des groupes alkyle en C₁₋₄, alcoxy en C₁₋₄ ou trifluorométhyle ;
Q² est un atome d'hydrogène, de fluor, de chlore, ou un groupe nitrile, hydroxy, alkyle en C₁₋₄, alcoxy en C₁₋₄, alkyle en C₁₋₄ substitué par jusqu'à 5 atomes de fluor, ou alcoxy en C₁₋₄ substitué par jusqu'à 5 atomes de fluor ;
Q³ est un atome d'hydrogène, de fluor, de chlore, ou un groupe méthyle, méthoxy, trifluorométhyle, difluorométhyle, trifluorométhoxy ou difluorométhoxy;
ou Q² et Q³ sont reliés pour former le résidu d'un cycle carbocyclique à 5, 6 ou 7 chaînons ;
R⁴ est H ou un groupe alkyle en C₁₋₄ ;
m vaut 0 ou 1 ;
n vaut 0, 1 ou 2 ; et
W est CH₂, CHF, CH(OH) ou CO.

2. Composé selon la revendication 1 dans lequel Ar représente un groupe benzisothiazol-3-yle ou benzothiophén-3-yle, chacun portant des groupes substituants R¹, R² et R³, et m et n sont tous deux nuls.

3. Composé selon la revendication 1 dans lequel Ar représente un groupe phényle portant des groupes substituants R¹, R² et R³, et m vaut 1 et n vaut O.

4. Composé selon la revendication 1 de formule IIA : 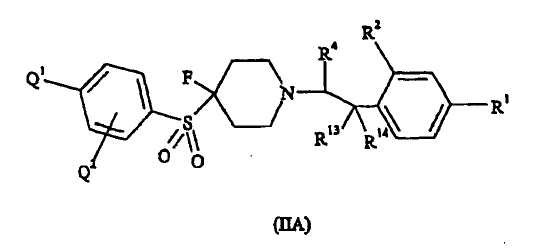 ou un sel pharmaceutiquement acceptable de ce composé,
formule dans laquelle R¹³ représente H et R¹⁴ représente H, F ou OH, ou R¹³ et R¹⁴ représentent ensemble un groupe céto ;
et Q¹, Q², R¹, R² et R⁴ sont tels que définis dans la revendication 1.

5. Composé selon l'une quelconque des revendications précédentes dans lequel Q¹ est choisi parmi H, F, Cl, Br, CN, carboxamide, hétéroaryle à 5 chaînons et NQ⁵Q⁶, où Q⁵ et Q⁶ complètent un cycle hétérocyclique ;
Q² est H, F ou Cl ;
Q³ est H ou F ;
R¹ est H, F, méthyle ou CF₃ ;
R² est H, F, méthyle ou CF₃ ; et
R³ est H.

6. Composition pharmaceutique comprenant un composé selon la revendication 1 et un véhicule pharmaceutiquement acceptable.

7. Composé selon la revendication 1 pour son utilisation dans un procédé de traitement du corps humain.

8. Utilisation d'un composé selon la revendication 1 pour fabriquer un médicament destiné au traitement ou à la prévention d'une affection où est impliquée l'activité des récepteurs 5-HT_{2A}.
